(19) Europäisches Patentamt
European Patent Office
Office européen des brevets

(11) EP 2 580 344 B1

(12) **EUROPEAN PATENT SPECIFICATION**

(45) Date of publication and mention
of the grant of the patent:
**05.11.2014 Bulletin 2014/45**

(51) Int Cl.:
***C12Q 1/37*** (2006.01)

(21) Application number: **11729135.1**

(22) Date of filing: **14.06.2011**

(86) International application number:
**PCT/GB2011/000888**

(87) International publication number:
**WO 2011/157982 (22.12.2011 Gazette 2011/51)**

(54) **FLUORESCENT-LABELLED DIUBIQUITIN SUBSTRATE FOR A DEUBIQUITINASE ASSAY**

FLUORESZENZMARKIERTES DIUBIQUITINSUBSTRAT FÜR EINEN DEUBIQUITINASETEST

SUBSTRAT DI-UBIQUITINE MARQUÉ PAR FLUORESCENCE POUR LE DOSAGE DE LA DÉ-UBIQUITINASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.06.2010 GB 201009941**

(43) Date of publication of application:
**17.04.2013 Bulletin 2013/16**

(73) Proprietor: **Medical Research Council
London WC1H 9LT (GB)**

(72) Inventors:
• **KOMANDER, David
Canbridge CB1 8DT (GB)**
• **YE, Yu
Cambridge CB2 1TP (GB)**

(74) Representative: **Maschio, Antonio
Maschio & Soames IP Limited
20 Carlton Crescent
Southampton, SO15 2ET (GB)**

(56) References cited:
**US-A1- 2007 292 907**

• **TIRAT A ET AL: "Synthesis and characterization of fluorescent ubiquitin derivatives as highly sensitive substrates for the deubiquitinating enzymes UCH-L3 and USP-2", ANALYTICAL BIOCHEMISTRY, ACADEMIC PRESS INC, NEW YORK, vol. 343, no. 2, 15 August 2005 (2005-08-15), pages 244-255, XP027219254, ISSN: 0003-2697 [retrieved on 2005-07-29]**
• **SATPAL VIRDEE ET AL: "Engineered diubiquitin synthesis reveals Lys29-isopeptide specificity of an OTU deubiquitinase", NATURE CHEMICAL BIOLOGY, vol. 6, no. 10, 1 October 2010 (2010-10-01), pages 750-757, XP55002769, ISSN: 1552-4450, DOI: 10.1038/nchembio.426**

## Description

The present invention relates to an assay for deubiquitinase (DUB) activity. In particular, the invention relates to an assay that uses a natural substrate for DUB activity, which comprises a labelled diubiquitin construct which is cleaved by the DUB enzyme. The invention also relates to methods for preparing such constructs, and techniques which make use of the assay.

Protein ubiquitination is a versatile posttranslational modification with roles in protein degradation, cell signaling, intracellular trafficking and the DNA damage response (Chen and Sun, Mol Cell 33 (3), 275-286, 2009; Komander, Biochem Soc Trans 37 (Pt 5), 937-953, 2009). Ubiquitin polymers are linked through one of seven internal lysine (K) residues or through the N-terminal amino group. Importantly, the type of ubiquitin linkage determines the functional outcome of the modification (Komander, 2009). The beststudied ubiquitin polymers, K48- and K63-linked chains, have degradative and non-degradative roles, respectively (Chen and Sun, 2009; Hershko and Ciechanover, Annu Rev Biochem 67, 425-479, 1998). However, recent data has revealed an unexpected high abundance of so-called atypical ubiquitin chains; for example, K11 linkages have been found to be as abundant as K48-linkages in S. *cerevisiae* (Peng et al., Nat Biotechnol 21 (8), 921-926, 2003; Xu et al., Cell 137 (1), 133-145, 2009).

Polyubiquitin chains are assembled on substrates through the concerted action of a three-step enzymatic cascade, involving an E1 ubiquitin activating enzyme, an E2 ubiquitin conjugating enzyme, and E3 ubiquitin ligases. While E3 ligases attach polyubiquitin chains to a target and thus confer substrate specificity, E2 enzymes are thought to determine the type of chain linkage in polyubiquitin chains. K48- and K63-specific E2 enzymes have been identified (Chen and Pickart, J Biol Chem 265, 21835-42, 1990; Hofmann and Pickart, Cell 96, 645-53, 1999), which allowed structural analysis of these chain types as well as a detailed understanding of specificity of ubiquitin binding domains (UBDs) and deubiquitinases (DUBs) (reviewed in Komander, 2009). K11-specific enzymes have been engineered, and are described in our copending UK patent application 1007704.8; see also Bremm et al., Nature Struct Mol Biol. 2010, Aug 17(8), 939-47. Moreover, N-terminally linked linear ubiquitin polymers can be synthesized enzymatically via the LUBAC complex (Kirisako et al, EMBO J 25(20):4877-87, 2006), or by molecular biology techniques.

Assays for DUB activity are known in the art, which can follow DUB activity in vitro. These assays are summarised in Shanmugham and Ovaa, Curr. Opin. Drug Disc. Dev. (2008) 11(5), 688-696. The most commonly used assay relies on the synthetic substrate Ubiquitin-7-amido-4-methylcoumarin (Ub-AMC), which is accepted by a variety of DUB enzymes. However, in common with other known synthetic substrate, Ub-AMC comprises a single ubiquitin molecule that has been labelled; the label is released upon cleavage by DUB. The cleavage, therefore, is not of an isopeptide bond between two ubiquitin moieties as occurring in a natural substrate. The released AMC molecule is fluorescent, and its rate of release can be measured and related to DUB activity (Dang et al, Biochemstry Feb 17;37(7):1868-79, 1998).

US2007292907 discloses compositions and methods for regulating ubiquitin-specific processing proteases by using fluorescently labelled polyubiquinated substrates.

There remains, therefore, a need in the art for an assay for DUB enzymes, which measures the cleavage of a natural ubiquitin linkage. Advantageously, such an assay would enable the use of ubiquitin dimers with particular linkages, to better reflect linkage specificity in DUB enzymes.

## Summary of the Invention

We have found that an assay for DUB enzyme activity can be based on a substrate which comprises a fluorescently labelled diubiquitin molecule, wherein cleavage of the diubiquitin molecule can be followed by fluorescence anisotropy, also referred to as fluorescence polarisation, or by Förster Resonance Energy transfer (FRET) In accordance with a first aspect of the invention, therefore, there is provided a substrate for measuring the activity of a deubiquitinating enzyme (DUB)as defined in claim 1.

Fluorescence anisotropy measures the tumbling of a fluorescent molecule in solution, and the tumbling rate depends on size (molecular weight) and shape of the molecule. Upon excitation of a fluorophore with polarized light, the degree of polarization in the emitted polarized light relates to the tumbling rate of the fluorescent molecule.

If the molecule changes in size, such as when a diubiquitin molecule is cleaved by DUB, the rate of tumbling will change; a reduction in size of the molecule by cleavage will change the tumbling rate, and change the degree of polarization in the emitted light, which can be readily measured.

The substrate is a labelled diubiquitin molecule as defined in the claims. Although ubiquitin dimers are preferred, longer polymers of ubiquitin can be used, especially in connection with DUB enzymes which do not cleave dimers effectively. If longer polymers are used, the label is preferably located on a terminal ubiquitin monomer; however, the fluorophore can be located on any monomer in the polyubiquitin molecule.

Advantageously, the C-terminal ubiquitin monomer is labelled.

In a further embodiment, more than one ubiquitin monomer may be labelled. For example, two monomers may be labelled using different dyes, and the resulting FRET emission monitored. The FRET signal is dependent on the proximity of the dyes, and will alter if the monomers are moved closer together or further apart.

The label may be any fluorescent label. Typically, fluorescent labels comprise a fluorophore, such as amine reactive isothiocyanate derivatives such as FITC and TRITC (derivatives of fluorescein and rhodamine), amine reactive succinimidyl esters such as NHS-fluorescein, sulfhydryl reactive maleimide activated fluororphores such as fluorescein-5-maleimide, and commercially-available fluorophores such as the Alexa dyes (Invitrogen).

Such compounds may be used to label an ubiquitin molecule, optionally by attachment via a polypeptide tag attached to the ubiquitin itself. For example, tags containing one or more cysteine residues can be labelled in a variety of ways. Biarsenical fluorescent labels are known in the art, and are useful in the present invention. Advantageously, the FlAsH tag fluorescent labelling system, available from Invitrogen, is employed. The peptide (X)CCXXCC replaced the C-terminal amino acids of the ubiquitin to be labeled, and the Lumio Green reagent (Invitrogen) used to label the molecule through interaction with the four cysteine residues. For example, the peptide WCCPGCC may be used.

The last 5 amino acids ($R^{72}LRGG^{76}$) of the ubiquitin C-terminus are replaced with the above sequence, WCCPGCC. Gly76 of the Ub C-terminal tail is replaced, in order to prevent the DUBs from cleaving the fluorescent tag from the ubiquitin molecule. A shorter deletion from the C-terminus of ubiquitin is permissible, as long as Gly76 is removed. Such a C-terminal replacement can only be made on the proximal ubiquitin. However, both distal and proximal ubiquitins can be labeled at their N-termini.

The addition of a Trp residue in the FlAsH tag has additional advantages allowing more accurate quantitation of the diubiquitin by measuring the absorbance at 280 nm. Ubiquitin does not contain Trp residues and it is hence challenging to measure its concentration accurately. However for kinetic measurements substrate concentrations need to be accurately determined and addition of a Trp residue allows this.

A tag can also comprise a single Cys residue, for labeling with Alexa fluorophores. Ubiquitin does not contain Cys residues, and hence incorporation of a Cys residue allows to site-specific labelling. The Cys residue may be preceding the N-terminus of ubiquitin. To generate a stable label at the C-terminus of Ub, the C-terminal Gly residue has to me mutated or removed, since otherwise the label would be released by DUB. The C-terminal residue Gly76 can be mutated to Cys to incorporate a label at the C-terminus of ubiquitin.

Polypeptide fluorophores, such as green fluorescent protein, yellow fluorescent protein or red fluorescent protein, may also be used; however, their larger size may reduce the sensitivity of the anisotropy assay.

The polarization anisotropy technique used in this aspect of the present invention has several advantages. It uses only a single label, which greatly facilitates the preparation of reagents for the assays, and measures the cleavage of the natural ubiquitin-ubiquitin isopeptide bond. This assay provides a better approximation to natural DUB activity than the methods of the prior art, and improves the measurement of enzyme kinetics for the deubiquitination reaction.

Fluorescently labeled diubiquitin can be prepared by fluorescently labeling an ubiquitin molecule which has been generated, either enzymatically for instance by treatment with a suitable E1 and E2 enzyme, or by means of chemical isopeptide ligation (see our copending UK patent application No 1007704.8) The linkage between the ubiquitin monomers is advantageously a K63, K48, K11 or linear link, meaning that the C-terminus of one ubiquitin monomer is linked to the K63, K48, K11 or Met 1 residue of another ubiquitin monomer. Other possible linkages include K6, K27, K29 and K33 linkages.

In an advantageous embodiment, a trimer, tetramer or other polymer of ubiquitin may be used. This is advantageous where, for example, the DUB to be assayed is inhibited by ubiquitin dimers.

In a second aspect, the invention provides a method for assaying the activity of a deubiquitinating enzyme (DUB) comprising exposing a substrate according to the first aspect of the invention to a DUB, and monitoring the cleavage of the substrate by measuring fluorescence polarisation anisotropy or FRET.

In one embodiment, the method employs an substrate wherein a single ubiquitin monomer is labeled, and a fluorescence polarization anisotropy measurement is take to detect cleavage of the substrate.

In a second embodiment, the method employs a substrate in which two ubiquitin monomers are labeled, and cleavage of the substrate is detected by changes in FRET.

Preferably, the enzyme kinetics of the DUB are assayed. The DUB may be a known DUB, or a candidate DUB. The method is suitable for identifying novel DUB activities. A kinetic analysis for a DUB takes only 30 to 60 minutes, and kinetic parameters for a DUB can be derived, e.g. Michaelis Menten parameters ($Km$) and catalytic rates ($kcat$).

If a second substrate is included in the assay, differences in the rate of cleavage of the first substrate can used to assess the relative specificity of the DUB to two different substrates.

If a second substrate is labeled with a different fluorophore that is excited at a different wavelength, a direct competition experiment against substrates can be performed. Such situation reflects the in vivo situation, where many linkages may be present, with an even greater degree of fidelity.

In a third aspect, the invention provides a method for assaying one or more candidate inhibitors of DUB activity. By measuring the kinetic parameters of the DUB reaction on the diubiquitin substrate of the first aspect the Michaelis constant (Km) of a substrate for any specific DUB can be derived. The influence of one or more inhibitors on the enzymatic reaction, either affecting binding of the DUB to the substrate (changing the Km), or affecting catalytic activity (changing the kcat), can therefore be measured. This provides a high-throughput technique for measuring the activity of candidate DUB inhibitors, for example in multiwell assay plates. Assays for DUB inhibitors have been described in the prior art, for example, in Shanmugham and Ovaa, Curr. Opin. Drug Disc. Dev. (2008) 11(5), 688-696; an assay according to the present invention, whilst configurable in a similar manner, has numerous advantages over the methods set forth in the art, for the reasons given above.

In a further embodiment of this aspect of the invention, the binding constants of a diubiqitin molecule to a DUB in which the catalytic activity has been abolished, for example by mutation of catalytic site residues, can be determined. The binding constant Kd may be directly related to the Michaelis constant Km. In this embodiment, inhibitors which are not dependent on the catalytic Cys residues may be identified, including allosteric inhibitors, such as inhibitors affecting the Kd of DUB to substrate. Such inhibitors inhibit the binding of the DUB to the labelled diUb. Binding of DUB to labelled diUb is responsible for the observed increase in anisotropy, which will be absent or reduced in the presence of an inhibitor. The assay of the present invention is thus compatible with high-throughput screening.

We have determined that this aspect of the invention may be practiced with FRET between two fluorophores located on each moiety of a labeled diUb, as well as fluorescence polarization anisotropy. On binding to their substrates, DUB enzymes open the conformation of the diubiquitin molecule prior to cleavage. Thus, binding of an inactive DUB to an ubiquitin dimer causes a change in FRET as the fluorophores are moved further apart.

In a fourth aspect of the invention, the assay of the invention may be used to identify and characterise ubiquitin binding domains (UBDs). For example, cell lysates may be screened for UBD presence by exposing different labelled diubiquitin constructs of the first aspect to the lysates and monitoring for changes in DUB activity in an assay according to the invention. Alternatively, proteins containing UBDs or isolated UBDs may be tested for binding to different labelled diubiquitin.

Moreover, the specificity of UBDs for any particular Ub chain linkage can be assessed, by comparing different labeled diUb reagents (f-diUb or FRET-diUb, linked via K6, K11, K27, K29, K33, K48, K63 or Met1) in a parallel determination of binding constants.

Accordingly, there is provided a method for assaying one or more candidate inhibitors of DUB activity, comprising the steps of:

(a) assaying a DUB according to the second aspect of the invention, to establish a reference activity for the DUB;
(b) assaying a DUB according step (a) in the presence of one or more candidate inhibitors of the DUB, and monitoring any changes in activity.

Preferably, the activity is selected from a binding activity and a cleavage activity. Fluorescence polarization anisotropy or FRET may be used.

Step (b) is advantageously performed in a multiple assay format, which allows assays to be conducted in parallel. The assay is preferably used in an HTS environment.

## Brief Description of the Figures

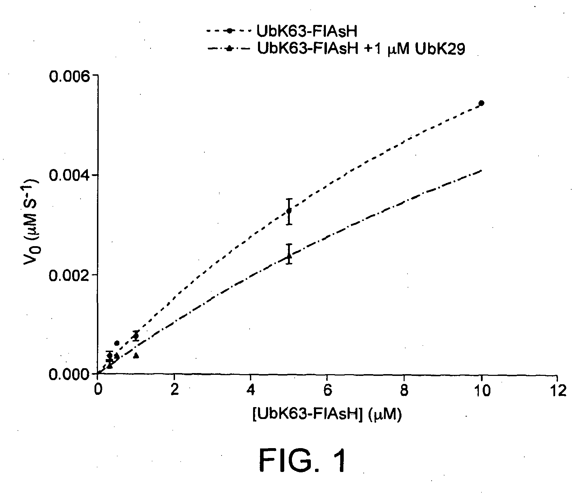

Figure 1: polarisation anisotropy plots showing determination of the cleavage of K63 linked substrate (UbK63) by the DUB TRABID at various concentrations of substrate.

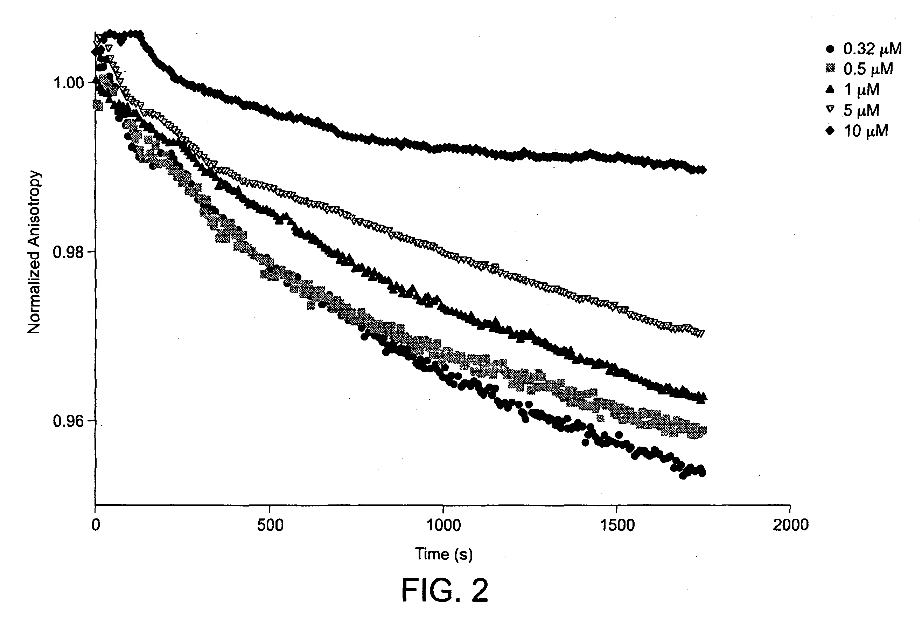

Figure 2: kinetic data calculated from the results shown in Fig 1. Plot shows confirmation of the TRABID:(Ub2K63 FlAsH) specificity constant and determination of the TRABID:(Ub2K29) $K_m$ by fluorescence polarization.

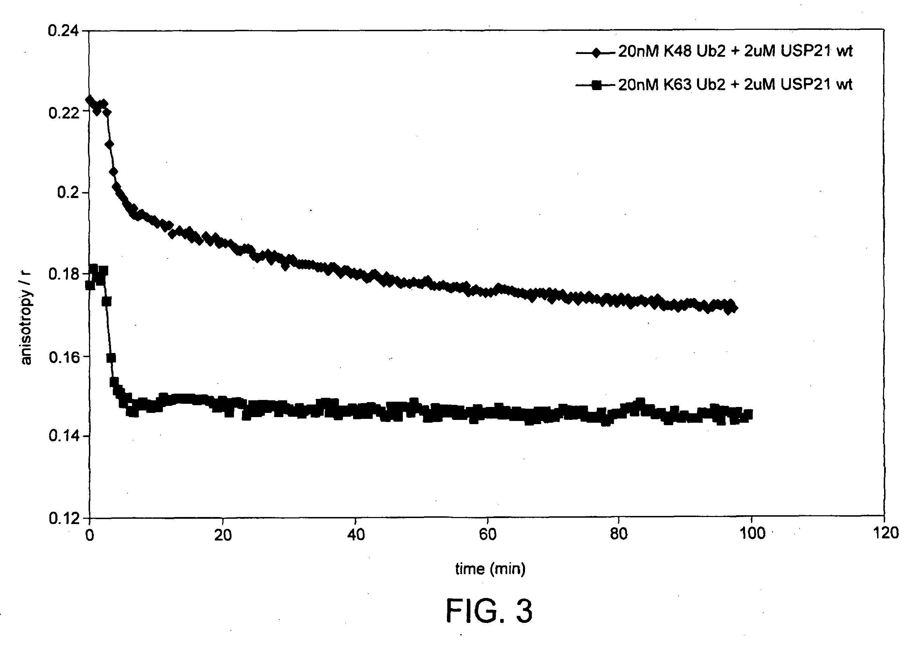

Figure 3: data obtained with USP21 enzyme, using K63 and K48 linked substrates.

Figure 4: Michaelis Menten kinetics for USP21, using fluorescent di-ubiquitin molecules made with K48, K63 or K11 linkages.

Figure 5: Michaelis Menten kinetics observed with vOTU DUB, using K63 and K48 linkages (A and B); AMSH DUB using K63 linked f-diUb (C); and OTUB1 DUB using K48 linked f-diUb (D).

Figure 6: Michaelis Menten kinetics measured using Ataxin-3 DUB and K63 (A) or K48 (B) linked f-diUb.

Figure 7: (A) A diagram showing possible interaction modes for di- and triUb (circles) in USP21 (space model). (B,C) Plots showing USP21WT and USP21 EEA (inactive) binding to (B) linear diUb-FlAsH and (C) triUb-FlAsH measured by fluorescence anisotropy. Error bars represent s.d. from mean.

## Detailed Description of the Invention

Unless defined otherwise, all technical and scientific terms used herein have the same meaning as commonly understood by those of ordinary skill in the art, such as in the arts of peptide chemistry, cell culture and phage display, nucleic acid chemistry and biochemistry. Standard techniques are used for molecular biology, genetic and biochemical methods (see Sambrook et al., Molecular Cloning: A Laboratory Manual, 3rd ed., 2001, Cold Spring Harbor Laboratory Press, Cold Spring Harbor, NY; Ausubel et al., Short Protocols in Molecular Biology (1999) 4th ed., John Wiley & Sons, Inc.).

DUB enzymes, as referred to herein, are deubiquitinating enzymes or deubiquitinases. In vivo, they can reverse the action of ubiquitin-conjugating enzymes by cleaving ubiquitin from target proteins. Over 100 DUBs are known in the human genome; see Reyes Turcu et al., (2009) Annual Review of Biochemistry 78:363-97, Komander et al, Nat Rev Mol Cell Biol 10(8), 560-563, 2009. DUBs are responsible for rescuing proteins from degradation, recycling or remodelling ubiquitin branches, regenerating free ubiquitin and the de novo production of ubiquitin, which is translated as a linear fusion protein containing multiple ubiquitin copies. Hence, DUBs regulate many processes that involve ubiquitination. Most DUBs are cysteine proteases, but some are metalloproteases, and various DUBs have been implicated in diseases, including cancer and neurodegeneration, and in both innate and adaptive immunity.

Fluorescent labelling includes any suitable technique for labelling polypeptides. For example, see the review provided in Zhang et al., Nat. Rev. Mol. Cell Biol. (2002) 3:906, and Marks & Nolan, Nature Methods (2006) 3:8, 591; Genger et al., Nature Methods (2008) 5:9, 763; Giepmans et al., Science (2006) 312 (5771): 217-224.

A preferred fluorescent label is the Fluorescein Arsenical Hairpin binding (FlAsH) labeling reagent, $EDT_2[4',5'$-bis(1,3,2-dithioarsolan-2-yl) fluorescein-(1,2-ethanedithiol)$_2]$. This is a bisarsenical compound that binds to polypeptides comprising the sequence, C-C-X-X-C-C, wherein "C" represents cysteine and "X" represents any amino acid other than cysteine (Griffin et al. Science 281:269-272, 1998). Adams et al. (Am Chem Soc. 124:6063-6076, 2002) have reported that the highest affinity is achieved when X-X is proline and glycine. FlAsH tags have been successfully incorporated at either the N-or C-termini of proteins, as well as exposed surface regions within a protein (Griffin et al., 1998; Adams et al., 2002, and Griffin et al. Methods Enzymol. 327:565-78, 2000). The bisarsenical dye is normally reacted with two ethylenedithiol (EDT) molecules for easier diffusion through the cell membrane. The FLASH-EDT$_2$ labeling reagent is non-fluorescent and becomes fluorescent upon binding to the "FLASH-tag" tetracysteine motif. When the FlAsH-EDT$_2$ dye is not bound to a protein, the small size of the EDT permits the free rotation of the arsenium atoms that quench the fluorescence of the fluorescein moiety. When a C-C-P-G-C-C labeled protein is mixed with the FlAsH-EDT2 dye, the arsenium atoms of the FlAsH dye react with the tetracysteine tag of the protein and form covalent bonds. The product of this reaction does not allow free rotation of the arsenium atoms and, because they no longer quench its fluorescence, the fluorescein moiety becomes fluorescent. The increase of the fluorescence is about 50,000 fold when the FlAsH dye is bound to protein (Griffin et al., 1988).

Other labels, such as commercial dyes, may also be used. The Alexa dyes produced by Invitrogen are examples of dyes useful in the practice of the invention (Panchuk-Voloshina et al., J Histochem Cytochem September 1, 1999 vol. 47 no. 9, 1179-1188).

Ubiquitin, as used herein, refers to ubiquitin and ubiquitin-like proteins. In one embodiment, it refers to ubiquitin specifically and excludes other ubiquitin-like proteins.

The linkage selected in a substrate according to the invention will depend on the specificity of the DUB to be assayed. Methods for preparing ubiquitin polymers using specific linkages are known in the art, for instance from Komander, D., et al., (2008) Mol. Cell 29, 451-464; Pickart, C.M. and Raasi, S. (2005) Methods Enzymol. 399, 21-36; Trempe, J.F., et al., (2005) EMBO J. 24, 3178-3189; and Bremm et al, Nature Struct Mol Biol. 2010, Aug; 17(8); 939-47.

In mammals there are about 100 DUBs categorized into five gene families: the ubiquitin C-terminal hydrolases (UCHs); the ubiquitin-specific peptidases (USPs/UBPs); the ovarian tumor (OTU) domain proteins; the Josephin or Machado-Joseph disease (MJD) proteins and the JAMM (Jab1/MPN domain-associated metalloisopeptidase) domain proteins. The first four families are cysteine peptidases, while the JAMM proteins are zinc metalloisopeptidase s. These DUB families have been the subjects of recent reviews [Amerik & Hochstrasser, Biochim Biophys Acta 2004, 1695:189-207; Soboleva & Baker, Curr Protein Pept Sci 2004, 5:191-200; Nijman et al., Cell 2005, 123:773-786; Reyes Turcu et al., Annu. Rev. Bioche. 2009, 78:363-397; Komander et al., Nat Rev Mol Cell Biol 2009, 10(8), 560-563.

Most DUBs contain a catalytic domain, and unrelated sequences either N-terminal or C-terminal (or both) to the catalytic domain. These flanking sequences have been shown to mediate substrate binding in a few cases.

Since most DUBs have been identified only by means of sequence similarity to catalytic motifs, there is limited functional information on many of these enzymes. However, the examples where functional insights have been gained indicate that DUBs can play crucial regulatory roles in the ubiquitin proteasome system (UPS), making them ideal drug target candidates for therapeutic intervention in UPS-related diseases.

DUBs include, but are not limited to, Isopeptidase T, Rpn11/POH1, UCH37, Ubp6/Usp14, Ubp8/Usp22, Ubp10, Usp16/Ubp-M, Usp21, 2A-DUB, Usp28, Usp44, Usp1, Usp11, Usp3, A20, CYLD, Usp15, Usp9Y, Doa4/Usp8, AMSH and Usp9X. For a complete list of DUBs discovered to date, see Komander et al., Nat Rev Mol Cell Biol., 2009.

Fluorescence polarization is known in the art. For example, see Principles of Fluorescence Spectroscopy, Third Edition, Joseph R. Lakowicz, ISBN-13: 978-0387-31278-1, Springer, New York, 2006; Gradinaru et al., Analyst, 2010, 135:452 - 459; and Huang & Aulabaugh, Methods in Molecular Biology 565, 2009, 127-143.

**Examples**

**Example 1: Analysis of diUb cleavage by OTU DUB TRABID**

The Ub-W-FlAsH construct was amplified by PCR from a plasmid encoding wild type ubiquitin with reverse primers, introducing the amino acid sequence WCCPGCC starting from residue 72 in the ubiquitin sequence, therefore replacing the last 5 residues in ubiquitin (RLRGG). Ubiquitin does not contain a Trp residue, resulting in low absorbance at OD280, complicating kinetic measurements, and incorporation of Trp together with the required CCXXCC sequence allows accurate quantitation of concentrations.

The PCR product was subsequently cloned into pET17b vector (available from EMD chemicals) using conventional methods. The expression and purification of this ubiquitin construct were done according to established methods (Pickart and Raasi, 2005). The overall yield is 10mg per litre. The ligation of K63-linked diubiquitin is carried out using equimolar amounts of UbWFlAsH, which can only form the proximal ubiquitin constituent and UbK63R, which can only be the distal ubiquitin. The methodology for ligation and subsequent purification of diubiquitin has been described elsewhere (Komander et al., EMBO Reports, 2009). The purified K63-linked diubiquitin, was labelled in buffer 1 (0.1% 2-Mercaptoethanol, 50mM Tris pH7.6), with Lumio Green reagent (Invitrogen) with equimolar ratio in room temperature for 1h, followed by overnight dialysis against buffer 1 (0.1% 2-Mercaptoethanol, 50mM Tris pH7.6). The concentration of FlAsH-labelled diubiquitin was determined using NanoDrop, and the efficiency of labeling by comparing the calculated absorptions at 280nm and 480nm. The efficiency of labeling was virtually 100%.

Hydrolysis reactions were initiated by adding 10 $\mu$L of enzyme preactivated in buffer 2 (50 mM Tris pH 7.6, 50 mM NaCl, 5 mM 2-mercaptoethanol, 1 $\mu$M TRABID) to 10 $\mu$L of fluorescent substrate in buffer 3 (50 mM Tris pH 7.6, 50 mM NaCl, 5 mM 2-mercaptoethanol, 0.6 - 20 $\mu$M (UbK63 FlAsH)$_2$). Three reactions were carried out at a time in parallel in a low-volume 384 well plate #3676 (Corning). Decrease in fluorescence anisotropy was then immediately monitored using a PHERAstar microplate reader (BMG Labtech) with a 485/520A/520B FP module. Data were collected over 29 minutes with an interval of 7 seconds. Each substrate concentration was carried out in duplicate or triplicate. The experiment was then repeated in the presence of 1 $\mu$M K29-linked diubiquitin, which was not fluorescently labeled and competes in the reaction with the labeled K63-linked diubiquitin. Progress curves were fitted to a single exponential decay function and the anisotropy signal was normalized to a $\mu$M concentration scale. Initial rates were determined by calculating the first derivative of the fitted progress data at t = 0. Initial rate values, v0, were fitted to the Michaelis-Menten equation, and kinetics analysed as described below.

*Characterizing the kinetics of deubiquitinases*

Confirmation of the TRABID:(UbK63 FlAsH)$_2$ specificity constant and determination of the TRABID:Ub2K29 Km by fluorescence polarization.

The initial rate of (UbK63 FlAsH)$_2$ cleavage was determined by following the decrease in anisotropy upon mixing TRABID (500 nM) and increasing concentrations of (UbK63 FlAsH)$_2$ in buffer (50 mM Tris pH 7.6, 50 mM NaCl, 5 mM 2-mercaptoethanol 20 $\mu$L, at 25 °C). The initial rates were plotted and data fitted to the Michaelis-Menten equation:

$$v_0 = \frac{V_{max}[S]}{K_m + [S]}$$

where $v_0$ is the initial rate, Vmax is the maximum velocity, [S] is substrate concentration and Km is the Michaelis constant.

This gives a kcat of 0.029 ($\pm$0.009) s$^{-1}$, a Km of 17 ($\pm$7) microM. This Km is very much higher than the substrate concentration (250 nM) obtained for Ub2K63 by western blotting to determine a specificity constant for TRABID cleavage, and validates the use of this approach. The data also give a specificity constant of 1.7 x103 ($\pm$2.0) M$^{-1}$s$^{-1}$ which is comparable to that determined by quantitative western blot, 2.5 ($\pm$ 0.4) x103 M$^{-1}$s$^{-1}$.)

The Km for TRABID: Ub2K29 was estimated from the initial rates of (UbK63 FlAsH)$_2$ cleavage in the presence of 1 microM Ub2K29 using the equation:

$$v_{0(K63FlAsH)} = \frac{\dfrac{V_{max(K63FlAsH)}[K63FlAsH]}{K_{m(K63FlAsH)}}}{1 + \dfrac{[K63FlAsH]}{K_{m(K63FlAsH)}} + \dfrac{[K29]}{K_{m(K29)}}}$$

where v0(K63FlAsH) is the calculated initial rate, Vmax(K63FlAsH) and Km(K63FlAsH) are the maximum velocity and Michaelis constant determined for (Ub2K63 FlAsH), respectively, in the direct experiment, [K63FlAsH] is the concentration of (Ub2K63 FlAsH), [K29] is the concentration of Ub2K29 and Km(K29) is the Km for TRABID:Ub2K29.

This gives a Km for TRABID:Ub2K29 of 2 $\pm$ 1.6 microM. Which is greater than the concentration of substrate (250 nM) used for the cleavage of Ub2K29 by TRABID in quantitative western blots, validating the use of this method to determine the specificity constant for TRABID cleaving Ub2K29. Since the apparent specificity constant decreases as the Km approaches the substrate concentration our data may underestimate the Ub2K29 specificity constant while accurately reflecting the Ub2K63 specificity constant. TRABID's 40-fold preference for Ub2K29 over Ub2K63 is therefore conservative and may represent an underestimate.

**Example 2**

**Cleavage of diUb by different DUB enzymes**

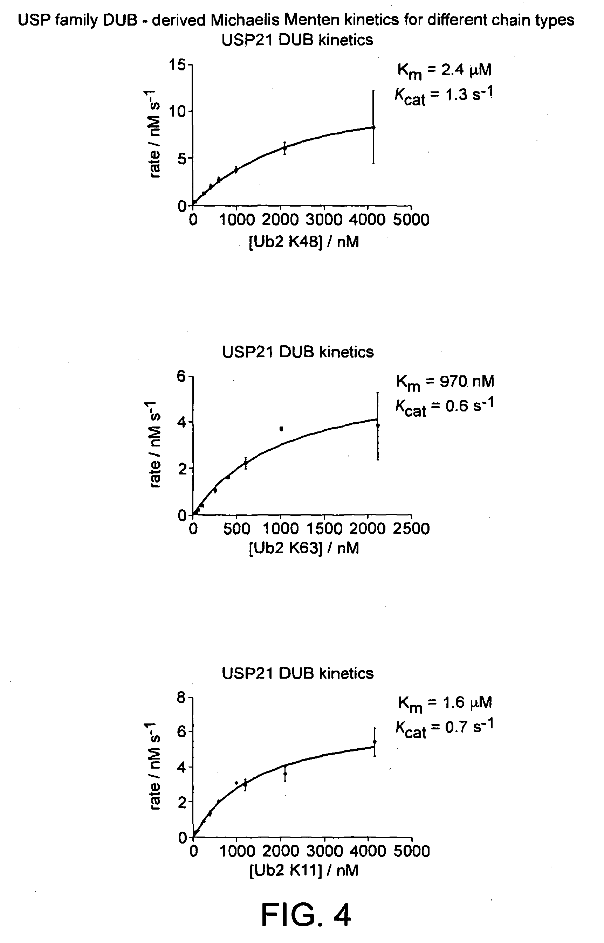

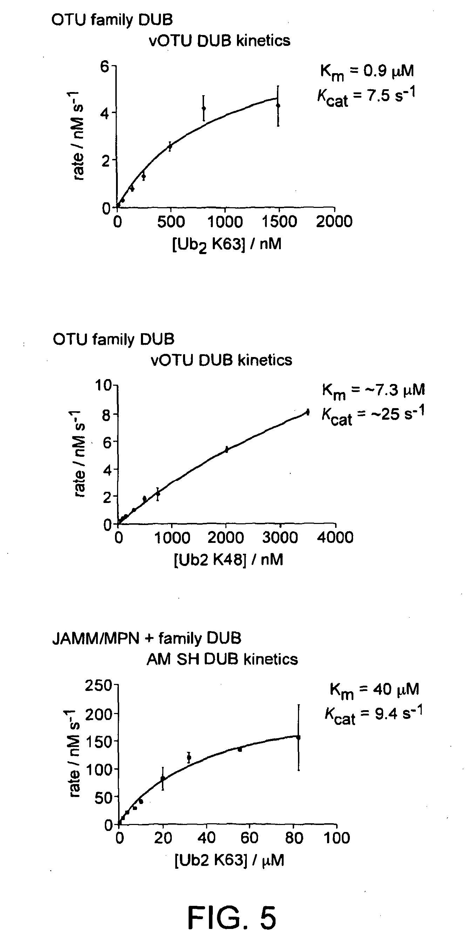

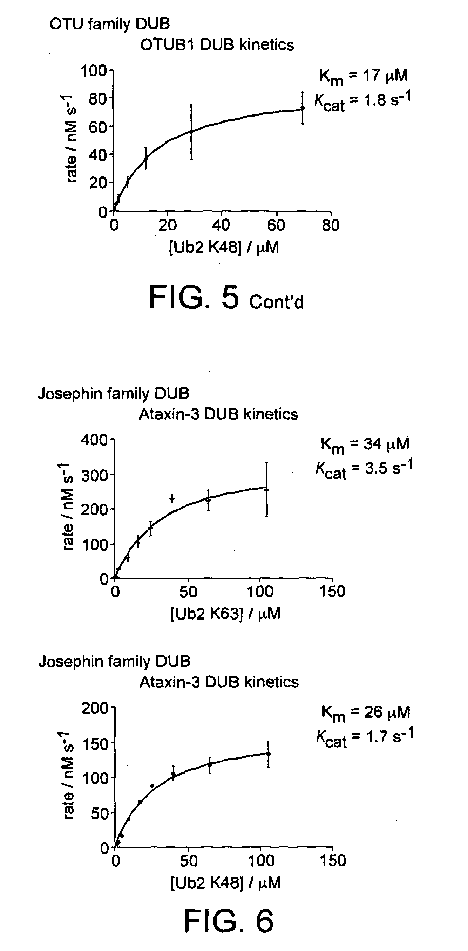

The experiments reported in Example 1 were repeated for further OTU domain DUBs (CCHFV viral OTU, OTUB1), a USP domain DUB (USP21), a JAMM/MPN+ domain DUB (AMSH) and a Josephin domain DUB (Ataxin3). The results are shown in Figures 4 to 7.

These experiments show not only that different types of DUB enzyme can be analysed by the method of the invention, but also that substrates having different chain specificities can be generated by conventional methodology and used in the DUB assay as described.

Diubiquitin molecules were generated with K48, K63 and K11 linkages, and used in an assay as described in Example 1. In each experiment, Michaelis Menten kinetics can be derived for the DUB enzyme, as shown in the accompanying figures.

**Example 3**

**DUB/UBD binding reagents**

Fluorescent diubiquitin (f-diUb) chains, as described in Examples 1 and 2, can be used as a binding reagent for DUB enzymes which have been inactivated by mutation (termed DUBi). The active site residues of DUBs are well established, and in most cases these enzymes are cysteine proteases that can be inactivating eg by mutation of the catalytic Cys residue. The inactive DUBi still binds to Ub chains.

Using f-diUb, binding constants for DUBi can be determined using fluorescence anisotropy/polarisation methods. Binding of DUBs to f-diUb leads to an increase in anisotropy, as the fluorescent molecule is now much larger than before.

We have established this assay in a 384 well format, using low amounts (5 nM) of f-diUb. We examined the binding of different fluorescent Ub chains to inactivated USP21i. As before, five residues of Ub at the C-terminus were replaced with a FlAsH-tag sequence preceded by Trp (WCCPGCC), which can be labelled by fluorescein derivatives.

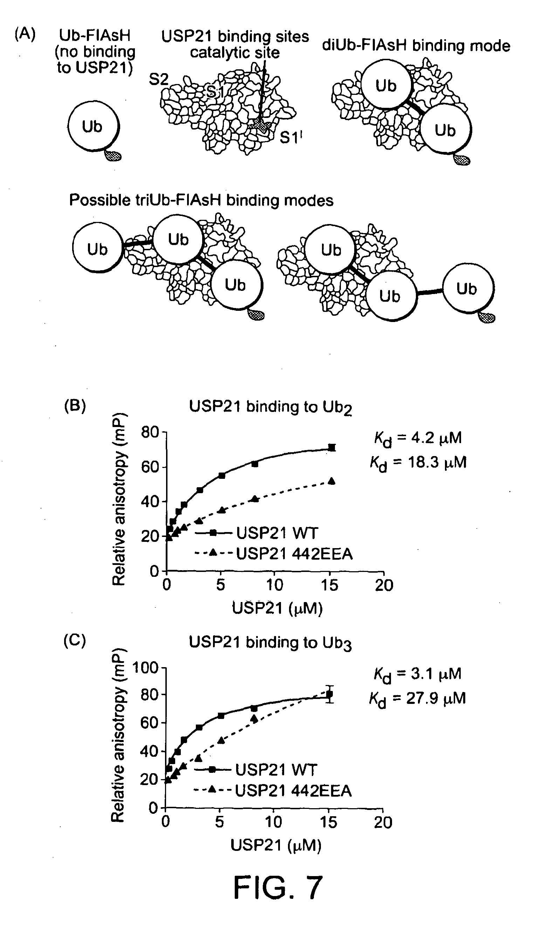

A PheraStar FS plate reader was used in the binding assay. Fluorescently labelled linear ubiquitin chains were diluted to 80 nM in FlAsH buffer (50 mM Tris, 50 mM NaCl, 0.1% $\beta$-mercaptoethanol, pH 7.6). Wild-type or mutant USP21i were serially diluted in FlAsH buffer to the indicated concentration range (Fig. 7C, D). 10 $\mu$l of the fluorescent Ub chain was mixed with equal volume of USP21i at different concentrations and incubated in room temperature for 1 h before measurement. Fluorescence anisotropy was measured in 384 well format employing a Pherastar FS plate reader, using a fluorescence polarisation module with excitation and emission wavelengths at 485 nm and 520 nm respectively. A control was used for either linear di- or triUb molecules where 10 $\mu$l of FlAsH buffer was added instead. This control was also used for the normalization of anisotropy reading. All binding assays were performed in triplicate.

Fluorescently labelled monoUb does not bind to the S1 Ub-binding site of USP21i, presumably because the bulky fluorescent group does not fit the active-site groove. By contrast, a linear diUb with this sequence added to the proximal moiety can bind to the S1 and S10 sites and a linear triUb can bind to the S2, S1 and S10 sites of the enzyme (Fig 7A). Linear triUb could also only interact with the S1/S10 sites, not benefitting from an S2 site (Fig 7A). Differences between di- and triUb binding therefore partly reflect a contribution of the S2 binding site. Anisotropy measurements revealed a small but reproducible difference between di- and triUb binding to USP21i, in which triUb bound with 1.4-fold higher affinity (Fig 7C, D). By contrast, the USP21 iEEA mutant bound to triUb with 1.5-fold lower affinity, compared with diUb (Fig 7C,D).

A similar approach is used to measure binding to ubiquitin binding domains (UBDs). Ubiquitination of proteins, which directs the proteins to the ubiquitin proteasome system, relies on binding of ubiquitin to proteins by means of a Ubiquitin binding domain (UBD) present on the proteins. Fluoresence anisotropy of the diubiquitin chain increases on binding by a UBD, since the size of the molecule increases. In the experiments reported above and in Fig 7, USP21i is functionally a UBD.

**Example 4**

**FRET Reagents; monitoring cleavage and binding activity**

**Methods**

Cloning

Donor Ub constructs were generated by PCR introducing an Ala-Cys sequence prior to the N-terminal Met1 of Ub. Ub mutants K11 R, K48R or K63Rwere used as template for PCR to generate a non-extendable donor Ub (Ala$_{-1}$Cys$_0$-UbKxR). The PCR product was subsequently cloned into the pOPINS[1]vector that harbors an N-terminal His$_6$-SUMO-tag, using the Infusion system (Clontech).

Acceptor Ub constructs used wild-type Ub sequence as template for PCR with primers introducing mutation G76C. Constructs intended as acceptor Ub were cloned into the pOPINE (Berrow, N. S. et al. Nucleic Acids Res35, e45, (2007)) vector that introduces a C-terminal KHHHHHH sequence (UbCys$_{76}$Lys$_{77}$). The vectors were transformed into Mach1 cells (Invitrogen) according to manufacturers protocols.

Cys-bearing linear diUb constructs were made by two subsequent rounds of site-directed mutagenesis using wild-type linear diUb construct in the pRS vector (Ye, Y. et al. EMBO reports 12, 350-357, (2011)), to introduce a Met-Ala-Cys sequence prior to Met1 of Ub at the N-terminus, and to introduce G152C mutation at the C-terminus. Mutagenesis was performed using the Quik-Change procedure (Stratagene) with KOD polymerase (Merck). The DNA product was subsequently digested with 1 μl DpnI for 1 h at 37°C and transformed into Mach1 cells. All constructs were confirmed by sequencing (Cogenics).

DUB constructs were described before; pOPINS-USP21 (196-565) (Ye, Y. et al. EMBO reports 12, 350-357, (2011)), pOPINK-vOTU (1-169) (Akutsu, M., et al. Proc Natl Acad Sci U S A 108, 2228-2233, (2011)), pET28-OTUB1(40-271) (Edelmann, M. J. et al. Biochem J 418, 379-390, (2009)) and pGEX6-AMSH (1-424) (McCullough, J., et al. J Cell Biol166, 487-492, (2004)). DUBs were inactivated by inactive by site-directed mutagenesis introducing C221A (USP21), C40A (vOTU), C91A (OTUB1) and E280A (AMSH), performed as stated above.

## Protein Expression and Purification

All constructs were transformed into Rosetta2 pLacl (DE3) cells, protein expression was induced with 1 mM IPTG at an OD$_{600}$ of 1.0, and cells were grown for 12-16 h at 20°C. Cells were pelleted and flash-frozen. Bacterial pellets expressing His-tagged proteins were re-suspended in buffer A (300 mM NaCl, 10 mM Imidazole, 50 mM Tris, pH 7.4). GST-tagged vOTU, OTUB1 and AMSH were re-suspended in lysis buffer (200 mM NaCl, 10 mM DTT, 25 mM Tris, pH 8.0). All cell suspensions were lysed by sonication and the cell lysate was cleared through centrifugation (30 min, 40000xg, 4°C).

Supernatant containing His-tagged proteins were loaded onto self-packed column containing 20 mL TALON resin (Clontech) followed by one-step elution using buffer B (50 mM Tris, 300 mM NaCl, 200 mM Imidazole, pH7.4). His-SUMO tagged proteins were cleaved overnight with recombinant SENP1 at 4°C. C-terminal His-tags were removed with carboxypeptidase A (Sigma). GST-tagged proteins were incubated with Glutathione-S-Sepharose 4B (GE Life Sciences) for 1 h under constant agitation at 4°C. The resin was subsequently washed with high salt buffer (25 mM Tris, 500 mM NaCl, 5 mM DTT, pH 8.5) and low salt buffer (20 mM Tris, 50 mM NaCl, 5 mM DTT, pH 8.5). C3 PreScission protease was used to cleaved off the GST-tag overnight at 4°C.

Ub proteins were further purified using ion exchange chromatography (MonoQ, GE Healthcare) and the peak fractions pooled and concentrated to >20 mg/ml concentration. DUBs were further purified by ion exchange chromatography using either ResourceQ or S (GE Healthcare). The peak fractions were concentrated to < 5 ml and further purified using gel filtration (Superdex75, GE Healthcare) in buffer C (phosphate buffer saline, pH7.4). The purity of all proteins were > 95% as judged on SDS-PAGE gel.

## Labeling and purification of Ubs

Alexa488 FluorC5 maleimide and Alexa647 FluorC2 maleimide were purchased from Invitrogen, dissolved in DMSO (1 mg/300 mL), snap-frozen in 20 mL aliquots and stored at -80 °C. Labeling of the Ub cysteine mutants was achieved by reaction of 80 mM Ub in 50 mM Tris, pH 7.2, 0.5 mM TCEP with 1.2 x excess of fluorophore dissolved in DMSO. The reaction mixture was agitated at room temperature in the dark for three hours. Unreacted dye was removed by size-exclusion chromatography (HiLoad S26/10 column, GE Healthcare) in elution buffer (50 mM Tris, pH 7.4), unreacted protein was separated from the desired product by anion-exchange chromatography (MonoQ 5/50, GE Healthcare) using elution buffer and applying a linear salt gradient from 0 to 1 M NaCl. Linear diUb comprising two Cys residues was labeled with a 1:1 mixture of each dye in DMSO following the same procedure.

Incorporation of dyes was confirmed using electrospray mass-spectrometry (ESI-MS).

## Ub Chain Ligation and Validation

The fluorescently labeled Ub mutants were assembled into diUb using described protocols. Ala$_{-1}$Cys$_0$*-UbK11R and UbCys$_{76}$*Lys$_{77}$ were assembled into K11NC using UBE2SΔC (Bremm, A., et al. Nat Struct Mol Biol 17, 939-947, (2010)) in presence of AMSH. Ala$_{-1}$Cys$_0$*-UbK63R and UbCys$_{76}$*Lys$_{77}$ were assembled into K63NC using Ubc13/Uev1a, and Ala$_{-1}$Cys$_0$*-UbK48R and UbCys$_{76}$*Lys$_{77}$ were assembled into K48NC using cdc34 (Komander, D. et al. Mol Cell 29, 451-464, (2008)).

Alternatively, Ala$_{-1}$Cys$_0$*-UbLys$_{77}$ was used as acceptor Ub to assemble NN-labeled diUb variants. In principle, Cys for labeling can be introduced at any other position in the Ub sequence.

Dual labeled diUb were separated from monoUb and single labeled diUb by anion exchange chromatography (MonoQ, GE Healthcare) as described above. In case of residual contamination, repeated runs of MonoQ were performed.

Dual labeled diUb was analyzed by tryptic digest MS/MS confirming specificity of the ligation reaction. Fluorescence signals were evaluated by SDS-PAGE and subsequent fluorescence scanning using a Typhoon fluorescence scanner at λ=526 and 670 nm.

**Anisotropy measurements**

Fluorescence anisotropy measurements were made using a 1 cm path length cuvette in a Cary Eclipse fluorimeter (Varian, Palo Alto, CA, USA). An excitation wavelength of linearly polarized light of 495 and 633 nm was used for excitation of donor or acceptor fluorophore, respectively using a band pass of 5 nm for both excitation and emission. Emissions were recorded at 515 and 651 nm. Anisotropy <r> is defined as

$$\langle r \rangle = \frac{I_{VV} - G * I_{VH}}{I_{VV} + 2G * I_{VH}} \quad (1)$$

where the subscripts of the light intensities $I$ define the position of the excitation (first subscript) and emission polarizers as being vertical V or horizontal $H$. G, the "G factor", is defined as

$$G = \frac{I_{HV}}{I_{HH}} \quad (2).$$

Sample concentrations were typically 3 - 500 nM. Anisotropy is concentration independent.

**Ensemble FRET measurements**

Ensemble FRET measurements were undertaken on a Cary Eclipse fluorimeter (Varian, Palo Alto, CA, USA) or a Pherastar plate-reader (BMGlabtech). An excitation wavelength of 488 nm was used to excite the donor fluorophore, using a band pass of 5 nm for both excitation and emission. Emissions were recorded between at 500 and 750 nm. FRET efficiency was determined

$$E = \frac{I_D * Q_D}{\left(I_D * Q_D + I_A * Q_A\right)} \quad (3)$$

where $I_A$ and $I_D$ are the fluorescence intensities and $Q_A$ and $Q_D$ the fluorescence quantum yields of acceptor and donor fluorophores, respectively. $I_A$ and $I_D$ were determined by integration of the fluorescence signal between 500 and 600 nm for $I_A$ and 620 and 750 nm for $I_D$, respectively. The quantum yield of Alexa 488, $I_D$, is 0.92 and 0.33 for Alexa 647, $I_A$ (source: Invitrogen).

**Quantitative FRET DUB assays**

The concentrations of FRET-diUb were established by Nanodropmeasurements using the fluorescence signal at 488 and 633 nm, determining the amount of labeled material, indicating that the majority of diUb comprised two labels.

The assays were performed using either Pherastar Plus or Pherastar FS plate reader in 384 well format. Fluorescence signals from Alexa 488 and Alexa 647 were monitored using a custom designed optic module with excitation wavelength at 485 nm and coincident emission detection at 520 nm and 675 nm. The fluorescence emission of 10 μL of FRET-diUb at 2x concentrations is measured prior to addition of active DUB.

DUB enzymes were serially diluted to 2x the given concentrations and 10 μl of enzymes was loaded into each well with FRET-diUb present using a multichannel pipette or the automatic sample loader from Pherastar FS. Loss in FRET was monitored over 40 min with cycle time of 4 s at fluorescence emission at 675 nm. The time gap between enzyme loading and the first fluorescence measurement of each well is 4 s on the Pherastar FS. We estimated the time gap between enzyme loading and fluorescence measurement using multichannel pipette to ~ 15 s. 20 μl of FRET-diUb at the lowest concentration used in the assay serves as negative control.

Emission signals at 675 nm were used for subsequent data analysis. The loss in fluorescence emission was plotted in Graphpad Prism and fitted to single and double exponential decays, from which residual plots were calculated. A double exponential decay curve fit was used when the value of the absolute sum of squares was reduced by at least 50 % compared to a single exponential decay. Where double-exponential fits were used, the individual decay curves of the fast and slow phases were extracted using Graphpad Prism, from the calculated Y0, plateau and rate constants (K), using the equation Y=(Y0 - Plateau)*exp(-K*X) + Plateau. Initial rates of reaction were calculated by differentiating the curves of the fast phase or the single-exponential decay curve. The value at the first time point was used for plotting the final Michaelis-Menten curve.

**Single-Molecule FRET measurements**

the instrumentation for single-molecule fluorescence TCCD and FRET has been reported in detail previously (Orte, A.; Clarke, R.; Balasubramanian, S.; Klenerman, D. Anal. Chem. 2006, 78, 7707-7715). To perform TCCD measurements, both lasers were used simultaneously, whereas for FRET experiments, the same instrument was used but without excitation at 633 nm. The sample conditions were 25 pM labeled Ub chain under native conditions (50 mM Tris buffer at pH 7.4, with 0.01% Tween20 to prevent glass adhesion) if not stated otherwise.

**Single-Molecule Fluorescence TCCD and FRET Data Analysis.**

For FRET experiments the proximity ratio histograms of all the time bins with acceptor intensities above 7 counts ms$^{-1}$ were analyzed (ACCEPTOR criterion). This approach filters out the zero peak, biasing the analysis to only significant FRET events. In order to build the histograms from FRET experiments, the proximity ratio is defined as

$$E = \frac{I_D}{\left(I_D + I_A * \gamma\right)} \quad (4)$$

where $I_A$ and $I_D$ are the fluorescence intensities in the acceptor and donor chanels, respectively. These intensities were corrected by the background autofluorescence (0.5-1.5 kHz) and the spectral crosstalk of the donor channel into the acceptor channel (around 3%) as well as the difference in detection efficiencies of the photon-multipliers in each channel quantified in the instrument constant $\gamma$.

The instrument constant $\gamma$ was found to be 0.54 and was determined by comparing the FRET efficiency of DNA-samples with known FRET efficiencies measured on the single molecule instrumentation with the measured efficiencies of a calibrated Cary400 fluorimeter.

For TCCD experiments, only the significant coincident events were analyzed; that is, the coincident events arising from chance were subtracted from the totals, following the methodology previously published (Orte, A.; Clarke, R.; Balasubramanian, S.; Klenerman, D. Anal. Chem. 2006, 78, 7707-7715). The parameter to study the coincidence levels is the association quotient, defined as

$$Q = \frac{r_S}{r_D + r_A - r_S} = \frac{r_C - r_E}{r_D + r_A - \left(r_C - r_E\right)} \quad (5)$$

where $r_S$ is the burst rate of the significant coincident events (chance coincident events, $r_E$, subtracted from the total coincident events, $r_C$), and $r_D$ and $r_A$ are the burst rates in the donor and acceptor channels, respectively. The association quotient is proportional to the fraction of dual-labeled molecules in solution.

Unlike FRET histograms, in TCCD experiments the histograms are built from the parameter Z, given by (Orte, A.; Clarke, R.; Balasubramanian, S.; Klenerman, D. Anal. Chem. 2006, 78, 7707-7715)

$$Z = \ln\left(\frac{I_A}{I_D}\right) \quad (6)$$

where $I_A$ and $I_D$ were as defined and corrected above.

A constrained fitting procedure was used for the TCCD histograms. (Ren, X.; Li, H.; Clarke, R. W.; Alves, D. A.; Ying, L.; Klenerman, D.; Balasubramanian, S. J. Am. Chem. Soc. 2006, 128, 4992-5000). Populations found in Z distributions were assumed to follow Gaussian functions. The center and the widths of the Gaussians are defined by the average brightness of the donor and acceptor fluorophores, $<I_D>$ and $<I_A>$, respectively. For each population, the center of the Gaussian is given by

$$x_c = \ln\left(\frac{\langle I_A \rangle}{\langle I_D \rangle}\right) \quad (7)$$

whereas the width of the Gaussian function is given by $k$ times the shot-limited width:

$$\sigma = K\sqrt{\frac{1}{\langle I_D \rangle} + \frac{1}{\langle I_A \rangle}} \quad (8)$$

In order to constrain the fitting of the TCCD histograms when multiple populations were detected, we assumed the brightness value of the donor fluorophore does not vary much in the two populations and this value equals the average brightness of the fluorophore during the measurement. Furthermore, the $k$ values are known, as obtained from dsDNA model samples. The TCCD histograms showed widths between 2.2- and 2.5-fold larger than the shot-noise limited width. Therefore, only three fitting parameters are used in this model: the acceptor fluorophore brightness values for the two populations and the relative fraction of the low-FRET species.

**Results**

To mechanistically understand the fundamental principles governing Ub chain interactions, we employed Förster resonance energy transfer (FRET) measurements. Alexa488 and Alexa647 Cys reactive dyes (Invitrogen) were attached to the C-terminus of a distal Ub, and to the N-terminus of a proximal Ub, and Lys63-(K63NC) and Lys48-linked (K48NC) diUb were generated using linkage specific enzymatic assembly. The photophysical properties of the dyes were unaffected in K48NC, while we observed slight quenching of Alexa488 in K63NC that was corrected for in subsequent measurements. FRET efficiency is inversely related to the distance between the fluorophore pair. K48NC and K63NC displayed robust FRET signals in ensemble measurements, with FRET efficiencies of 54% for K48NC and 27% for K63NC revealing that in the context of diUb, the fluorophores are in FRET distance. Incubation of labeled diUb with active DUBs of the ubiquitin specific protease (USP) or ovarian tumor (OTU) family, (human USP21, human OTUB1, or the viral OTU domain of Crimean Congo hemorrhagic fever virus (vOTU)) resulted in disappearance of the FRET signal, with kinetics similar to previously established diUb-based assay. This shows that the FRET reagents successfully monitor diUb cleavage, as the FRET signal disappears when the diUb molecule is cleaved into separate monomers.

To further determine the distinct chain conformations present in K48NC and K63NC, we employed single molecule fluorescent techniques. Labeled diUb species were measured at picomolar (pM) concentration under equilibrium conditions in a confocal laser microscopy setup. The total number of molecules containing the fluorophore pair regardless of whether a FRET signal is present, is assessed by two-color coincidence detection (TCCD), where coincident signals are recorded with simultaneous excitation at both donor and acceptor wavelengths (Orte, et al., Analytical chemistry 78, 7707-7715, (2006)). Subsequently, diUb populations presenting a FRET signal are recorded by excitation at the donor wavelength and subsequent detection of coincident signals at both donor and acceptor wavelengths, from the same sample under identical conditions as for TCCD. While comparison of TCCD and FRET reveals the proportion of molecules bearing FRET, the FRET histograms corresponding to individual molecules with similar FRET efficiencies can be fitted to Gaussian distributions, each representing a distinct conformation of diUb.

For K48NC, all molecules (100% compared to TCCD) displayed a FRET signal, which can be further separated in two distinct FRET populations. A high-FRET species (FRET efficiency E=0.69) represents ~85% of all molecules, while the remaining ~15% were represented by a low-FRET species (E=0.41). The distribution of FRET populations was consistent with the previously reported equilibrium ratio between compact structures derived from NMR RDC measurements. Hence the high-FRET species most likely corresponds to the main diUb conformation with shielded hydrophobic patches, whilst the low-FRET species likely corresponds to a conformation with partly exposed hydrophobic patches.

For K63NC, the majority (63%) of dual-labeled molecules did not display a FRET signal, consistent with an open conformation consistent with previous structural models. Surprisingly, a significant population (37%) of K63NC displayed a high FRET signal (E=0.50), indicating the presence of compact K63NC conformations. Such compact conformation(s) account for the observed robust FRET signal in ensemble measurements, yet they had not been observed by NMR (Varadan, et al. J Biol Chem 279, 7055-7063, (2004)).

The Ub chain populations observed in single molecule FRET represent conformationally stable domain orientations. The diffusion controlled residence time of a labeled diUb in the confocal volume is ~1 ms, suggesting that interconversion between conformations is >1 ms since we would otherwise observe an average conformation.

Understanding Ub chain conformations at the single molecule level allowed us to investigate whether ubiquitin interacting proteins (UbIPs) such as DUBs interact with available diUb conformations ('conformational selection'), or whether they remodel chains upon binding ('induced fit'). For this we incubated K63NC and K48NC at pM concentrations with unlabeled UbIPs at $\mu$M concentrations exceeding the measured $K_D$ of the interaction.

The crystal structure of Lys63-linked diUb in complex with a linkage specific antibody (pdb-id 3dvg, Newton, K. et al. Cell 134, 668-678, (2008)) represents the only structure of a Lys63-linked diUb in a compact conformation. Interestingly, incorporation of the antibody with K63NC increases the high-FRET relative to the non-FRET population. The observed equivalent FRET efficiency in the unbound and antibody-bound state (E=0.5), further suggests that the antibody selects the pre-existing compact K63NC conformation, in accordance with published models for antibody/antigen recognition (James, L. C., et al., Science 299, 1362-1367, (2003)).

DUBs access the isopeptide bond between Ub moieties to catalyze its hydrolysis. The only DUB-diUb complex structure reported to date shows how AMSH-LP, a member of the JAMM metalloprotease family, binds to the open conformation of Lys63-linked diUb (2znv, Sato, Y. et al. Nature 455, 358-362, (2008)). Accordingly, inactivated AMSH (denoted by suffix 'i', i.e. AMSHi) depleted the high-FRET and increased the non-FRET population of K63NC. USPs (the largest family of DUBs in humans with >50 members) and OTU DUBs (15 members in humans) have so far only been reported in complex with a 'distal' monoUb bound to the active site Cys residue (Komander, D., et al., Nat Rev Mol Cell Biol 10, 550-563, (2009)). These structures revealed that the C-terminal five residues of the distal Ub are stretched out by extensive interactions, presumably separating the Ub moieties to open conformations. Indeed, inactivated USP21i (Ye, Y. et al. EMBO reports 12, 350-357, (2011)), or vOTUi (Akutsu, M., et al., Proc Natl Acad Sci U S A 108, 2228-2233, (2011)) also depleted the high-FRET and increased a non-FRET population of K63NC, similar to AMSHi. We conclude that while the Lys63 linkage-specific antibody selects the closed conformations of K63NC, DUBs select the open conformation of K63NC. Thus the apparent binding mechanism of Lys63-linkages can be explained by conformational selection.

In contrast to K63NC, non-FRET open conformations could not be observed in K48NC. However, upon incubation of K48NC with USP21i vOTUi or with the inactivated K48-specific DUB OTUB1i (Edelmann, M. J. et al. Biochem J 418, 379-390, (2009)), loss of the FRET population indicated formation a novel non-FRET conformation of K48NC. The non-FRET conformation must be a result of DUB binding, as no quenching of dyes was detected in life-time measurements. Hence, DUBs remodel K48NC into open conformations similar to observations made for K63NC, in accordance with structural models. This implies that DUB interactions with Lys48 chains follows 'induced fit' mechanisms, indicating that DUBs 'open' Ub chains.

These experiments moreover demonstrate that FRET measurements can be used to follow the binding of proteins containing a UBD to polyubiquitin, since the UBDs open the conformation of the ubiquitin on binding, producing a detectable change of the fluorescent signal.

Various modifications and variations of the described aspects and embodiments of the present invention will be apparent to those skilled in the art without departing from the scope of the present invention. Although the present invention has been described in connection with specific preferred embodiments, it should be understood that the invention as claimed should not be unduly limited to such specific embodiments. Indeed, various modifications of the described modes for carrying out the invention which are apparent to those skilled in the art are intended to be within the scope of the following claims.

**Claims**

1. A substrate for measuring the activity of a deubiquitinating enzyme (DUB), comprising a diubiquitin molecule, wherein the C-terminal ubiquitin in the diubiquitin molecule is labeled with a fluorescent label, and wherein Gly76 in said C-terminal diubiquitin is replaced with a sequence to incorporate the fluorescent label.

2. A substrate according to claim 1, wherein a Trp residue is incorporated with the fluorescent label to allow accurate quantification.

3. A substrate according to any preceding claim, which comprises three or more ubiquitin monomers.

4. A substrate according to any preceding claim, wherein the fluorescent label is a biarsenical fluorescent reagent, preferably wherein the biarsenical reagent is $EDT_2$[4',5'-bis(1,3,2-dithioarsolan-2-yl) fluorescein-(1,2-ethanedithiol)$_2$].

5. A substrate according to any preceding claim, wherein at least two ubiquitin monomers are labeled with different fluorescent labels and wherein the different fluorescent labels optionally constitute a FRET pair.

6. A substrate according to any preceding claim, wherein each linkage between the ubiquitin monomers comprises a link between a lysine residue at the same position and the C-terminus of an adjacent monomer.

7. A substrate according to claim 6, wherein the lysine residue is selected from the group consisting of K6, K11, K27, K29, K33, K48 and K63, preferably selected from the group consisting of K63, K48 and K11.

8. A substrate according to any one of claims 1 to 6, wherein the ubiquitin monomers are linear, linked through Met 1.

9. A method for assaying the activity of a deubiquitinating enzyme (DUB) comprising exposing a substrate according to any one of claims 1 to 8 to a DUB, and monitoring the cleavage of the substrate by fluorescence anisotropy or FRET.

10. A method according to claim 9, wherein the enzyme kinetics of the DUB are assayed.

11. A method for assaying the binding activity of a UBD comprising exposing a substrate according to any one of claims 1 to 7 to a UBD which is an inactive DUB or a UBD which does not cleave a substrate according to any one of claims 1 to 7, and monitoring the binding of the substrate to the UBD by fluorescence anisotropy or FRET.

12. A method for assaying one or more candidate inhibitors of DUB activity, comprising the steps of:

    (a) assaying a DUB according to any one of claims 9 to 11, to establish a reference activity for the DUB;
    (b) assaying a DUB according step (a) in the presence of one or more candidate inhibitors of the DUB, and monitoring any changes in activity.

13. A method according to claim 12, wherein the activity is selected from a binding activity and a cleavage activity.

14. A method according to claim 12, in which step (b) is performed in a multiple assay format.

**Patentansprüche**

1. Substrat zum Messen der Aktivität eines deubiquitinierenden Enzyms (DUB), welches ein Diubiquitinmolekül umfasst, wobei das Ubiquitin am C-Terminus des Diubiquitinmoleküls mit einem fluoreszierenden Marker gekennzeichnet ist und wobei Gly75 am C-Terminus des Diubiquitins durch eine Sequenz ersetzt wird, um den fluoreszierenden Marker einzubinden.

2. Substrat nach Anspruch 1, wobei ein Trp-Rest im fluoreszierenden Marker eingebunden wird, um exaktes Quantifizieren zu ermöglichen.

3. Substrat nach einem der vorhergehenden Ansprüche, welches drei oder mehr Ubiquitinmonomere aufweist.

4. Substrat nach einem der vorhergehenden Ansprüche, wobei der fluoreszierende Marker ein Biarsen-Fluoreszensreagens ist, wobei das Biarsen-Fluoreszensreagens vorzugsweise EDT$_2$[4',5'-bis(1,3,2-dithioarsolan-2-yl)fluorescin-(1,2-ethandithiol)$_2$] ist.

5. Substrat nach einem der vorhergehenden Ansprüche, wobei wenigstens zwei Ubiquitinmonomere mit verschiedenen fluoreszierenden Markern gekennzeichnet sind und wobei die verschiedenen fluoreszierenden Marker optional ein FRET-Paar bilden.

6. Substrat nach einem der vorhergehenden Ansprüche, wobei jede Bindung zwischen den Ubiquitinmonomeren eine Bindung zwischen einem Lysinrest an derselben Position und dem C-Terminus eines angrenzenden Monomers umfasst.

7. Substrat nach Anspruch 6, wobei der Lysinrest ausgewählt ist aus der Gruppe bestehend aus K6, K11, K27, K29, K33, K48 und K63, vorzugsweise aus der Gruppe bestehend aus K63, K48 und K11.

8. Substrat nach einem der Ansprüche 1 bis 6, wobei die Ubiquitinmonomere linear und über Met 1 verbunden sind.

9. Verfahren zum Untersuchen der Aktivität eines deubiquitinierenden Enzyms (DUB), folgende Schritte umfassend: Aussetzen eines Substrats nach einem der Ansprüche 1 bis 8 einem DUB und Überwachen der Spaltung des Substrats unter Einsatz von Fluoreszenzanisotropie oder FRET.

10. Verfahren nach Anspruch 9, wobei die Enzymkinetik des DUB untersucht wird.

11. Verfahren zum Untersuchen der Bindungsaktivität einer UBD, folgende Schritte umfassend: Aussetzen eines Substrats nach einem der Ansprüche 1 bis 7 einer UBD, welche ein inaktives DUB ist oder eine UBD, welche ein Substrat nach einem der Ansprüche 1 bis 7 nicht spaltet, und Überwachen der Bindung des Substrats an die UBD unter Einsatz von Fluoreszenzanisotropie oder FRET.

12. Verfahren zum Untersuchen von einem oder mehreren Kandidateninhibitoren der DUB-Aktivität, folgende Schritte umfassend:

(a) Untersuchten eines DUB nach einem der Ansprüche 9 bis 11, um eine Referenzaktivität für das DUB festzulegen;
(b) Untersuchten eines DUB nach Schritt (a) in Gegenwert von einem oder mehreren Kandidateninhibitoren des DUB und Überwachen der Veränderungen in der Aktivität.

13. Verfahren nach Anspruch 12, wobei die Aktivität aus einer Bindungsaktivität und einer Spaltungsaktivität ausgewählt ist.

14. Verfahren nach Anspruch 12, wobei Schritt (b) in einem Multiassayformat ausgeführt wird.

**Revendications**

1. Un substrat pour mesurer l'activité d'une enzyme de désubiquitination (enzyme DUB), comprenant une molécule de diubiquitine, dans laquelle l'ubiquitine à terminaison C dans la molécule de diubiquitine est marquée avec un marqueur fluorescent, et dans laquelle le Gly76 dans ladite diubiquitine à terminaison C est remplacé par une séquence destinée à être intégrée au marqueur fluorescent.

2. Un substrat selon la revendication 1, dans lequel un résidu Trp est intégré au marqueur fluorescent pour permettre une quantification précise.

3. Un substrat selon toute revendication précédente, qui comprend au moins trois monomères d'ubiquitine.

4. Un substrat selon toute revendication précédente, dans lequel le marqueur fluorescent est un réactif fluorescent à deux atomes d'arsenic, et de préférence dans lequel le réactif à deux atomes d'arsenic est l'EDT$_2$[4',5'-bis(1,3,2-dithioarsolan-2-yl) fluorescéine-(1,2-éthanedithiol)$_2$].

5. Un substrat selon toute revendication précédente, dans lequel au moins deux monomères d'ubiquitine sont marqués avec des marqueurs fluorescents différents, et dans lequel ces marqueurs fluorescents différents constituent de manière facultative une paire FRET.

6. Un substrat selon toute revendication précédente, dans lequel chaque lien entre les monomères d'ubiquitine comprend une liaison entre un résidu de lysine à la même position et la terminaison C d'un monomère adjacent.

7. Un substrat selon la revendication 6, dans lequel le résidu de lysine est sélectionné parmi le groupe constitué de K6, K11, K27, K29, K33, K48 et K63, et de préférence sélectionné parmi le groupe constitué de K63, K48 et K11.

8. Un substrat selon toute revendication parmi les revendications 1 à 6, dans lequel les monomères d'ubiquitine sont linéaires et reliés par l'intermédiaire de Met 1.

9. Une méthode pour mesurer l'activité d'une enzyme de désubiquitination (DUB) comprenant le fait d'exposer un substrat selon toute revendication parmi les revendications 1 à 8 à une enzyme DUB, et la surveillance du clivage du substrat par anisotropie de fluorescence ou FRET.

10. Une méthode selon la revendication 9, dans laquelle la cinétique enzymatique de la DUB est analysée.

11. Une méthode pour mesurer l'activité de liaison d'un UBD comprenant l'exposition d'un substrat selon une quelconque des revendications 1 à 7 à un UBD qui est une DUB inactive ou un UBD qui ne clive pas un substrat selon une quelconque des revendications 1 à 7, et la surveillance de la liaison du substrat à l'UBD par anisotropie de fluorescence ou FRET.

12. Une méthode pour mesurer au moins un candidat inhibiteur de l'activité de l'enzyme DUB comprenant les étapes suivantes :

(a) mesurer une DUB selon une quelconque des revendications 9 à 11, afin d'établir une activité de référence pour la DUB ;
(b) mesurer une DUB selon l'étape (a) en présence d'un candidat inhibiteur de la DUB ou de plusieurs, et surveiller tout changement d'activité.

13. Une méthode selon la revendication 12, dans laquelle l'activité est sélectionnée parmi une activité de liaison et une activité de clivage.

14. Une méthode selon la revendication 12, dans laquelle l'étape (b) est exécutée dans un format d'essais multiples.

FIG. 1

FIG. 2

EP 2 580 344 B1

FIG. 3

EP 2 580 344 B1

USP family DUB - derived Michaelis Menten kinetics for different chain types

FIG. 4

OTU family DUB

### vOTU DUB kinetics

$K_m = 0.9 \ \mu M$

$K_{cat} = 7.5 \ s^{-1}$

OTU family DUB

### vOTU DUB kinetics

$K_m = {\sim}7.3 \ \mu M$

$K_{cat} = {\sim}25 \ s^{-1}$

JAMM/MPN + family DUB

### AM SH DUB kinetics

$K_m = 40 \ \mu M$

$K_{cat} = 9.4 \ s^{-1}$

## FIG. 5

OTU family DUB

OTUB1 DUB kinetics

$K_m = 17\ \mu M$

$K_{cat} = 1.8\ s^{-1}$

FIG. 5 Cont'd

Josephin family DUB

Ataxin-3 DUB kinetics

$K_m = 34\ \mu M$

$K_{cat} = 3.5\ s^{-1}$

Josephin family DUB

Ataxin-3 DUB kinetics

$K_m = 26\ \mu M$

$K_{cat} = 1.7\ s^{-1}$

FIG. 6

FIG. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 1007704 A
- US 2007292907 A
- US P21196565 B

### Non-patent literature cited in the description

- CHEN ; SUN. *Mol Cell,* 2009, vol. 33 (3), 275-286
- KOMANDER. *Biochem Soc Trans,* 2009, vol. 37, 937-953
- HERSHKO ; CIECHANOVER. *Annu Rev Biochem,* 1998, vol. 67, 425-479
- PENG et al. *Nat Biotechnol,* 2003, vol. 21 (8), 921-926
- XU et al. *Cell,* 2009, vol. 137 (1), 133-145
- CHEN ; PICKART. *J Biol Chem,* 1990, vol. 265, 21835-42
- HOFMANN ; PICKART. *Cell,* 1999, vol. 96, 645-53
- BREMM et al. *Nature Struct Mol Biol.,* August 2010, vol. 17 (8), 939-47
- KIRISAKO et al. *EMBO J,* 2006, vol. 25 (20), 4877-87
- SHANMUGHAM ; OVAA. *Curr. Opin. Drug Disc. Dev.,* 2008, vol. 11 (5), 688-696
- DANG et al. *Biochemstry,* 17 February 1998, vol. 37 (7), 1868-79
- SAMBROOK et al. Molecular Cloning: A Laboratory Manual. Cold Spring Harbor Laboratory Press, 2001
- AUSUBEL et al. Short Protocols in Molecular Biology. John Wiley & Sons, Inc, 1999
- REYES TURCU et al. *Annual Review of Biochemistry,* 2009, vol. 78, 363-97
- KOMANDER et al. *Nat Rev Mol Cell Biol,* 2009, vol. 10 (8), 560-563
- ZHANG et al. *Nat. Rev. Mol. Cell Biol.,* 2002, vol. 3, 906
- MARKS ; NOLAN. *Nature Methods,* 2006, vol. 8, 591
- GENGER et al. *Nature Methods,* 2008, vol. 9, 763
- GIEPMANS et al. *Science,* 2006, vol. 312 (5771), 217-224
- GRIFFIN et al. *Science,* 1998, vol. 281, 269-272
- ADAMS et al. *Am Chem Soc.,* 2002, vol. 124, 6063-6076
- GRIFFIN et al. *Methods Enzymol.,* 2000, vol. 327, 565-78
- PANCHUK-VOLOSHINA et al. *J Histochem Cytochem,* 01 September 1999, vol. 47 (9), 1179-1188
- KOMANDER, D. et al. *Mol. Cell,* 2008, vol. 29, 451-464
- PICKART, C.M. ; RAASI, S. *Methods Enzymol.,* 2005, vol. 399, 21-36
- TREMPE, J.F. et al. *EMBO J.,* 2005, vol. 24, 3178-3189
- AMERIK ; HOCHSTRASSER. *Biochim Biophys Acta,* 2004, vol. 1695, 189-207
- SOBOLEVA ; BAKER. *Curr Protein Pept Sci,* 2004, vol. 5, 191-200
- NIJMAN et al. *Cell,* 2005, vol. 123, 773-786
- REYES TURCU et al. *Annu. Rev. Bioche.,* 2009, vol. 78, 363-397
- KOMANDER et al. *Nat Rev Mol Cell Biol.,* 2009
- JOSEPH R. LAKOWICZ. Principles of Fluorescence Spectroscopy. Springer, 2006
- GRADINARU et al. *Analyst,* 2010, vol. 135, 452-459
- HUANG ; AULABAUGH. *Methods in Molecular Biology,* 2009, vol. 565, 127-143
- BERROW, N. S. et al. *Nucleic Acids Res,* 2007, vol. 35, e45
- YE, Y. et al. *EMBO reports,* 2011, vol. 12, 350-357
- AKUTSU, M. et al. *Proc Natl Acad Sci U S A,* 2011, vol. 108, 2228-2233
- EDELMANN, M. J. et al. *Biochem J,* 2009, vol. 418, 379-390
- MCCULLOUGH, J. et al. *J Cell Biol,* 2004, vol. 166, 487-492
- BREMM, A. et al. *Nat Struct Mol Biol,* 2010, vol. 17, 939-947
- KOMANDER, D. et al. *Mol Cell,* 2008, vol. 29, 451-464
- ORTE, A. ; CLARKE, R. ; BALASUBRAMANIAN, S. ; KLENERMAN, D. *Anal. Chem.,* 2006, vol. 78, 7707-7715

- **REN, X. ; LI, H. ; CLARKE, R. W. ; ALVES, D. A. ; YING, L. ; KLENERMAN, D. ; BALASUBRAMANIAN, S.** *J. Am. Chem. Soc.,* 2006, vol. 128, 4992-5000
- **ORTE et al.** *Analytical chemistry,* 2006, vol. 78, 7707-7715
- **VARADAN et al.** *J Biol Chem,* 2004, vol. 279, 7055-7063
- **NEWTON, K. et al.** *Cell,* 2008, vol. 134, 668-678
- **JAMES, L. C. et al.** *Science,* 2003, vol. 299, 1362-1367
- **2ZNV, SATO, Y. et al.** *Nature,* 2008, vol. 455, 358-362
- **KOMANDER, D. et al.** *Nat Rev Mol Cell Biol,* 2009, vol. 10, 550-563